# EUROPÄISCHE PATENTSCHRIFT

(11) **EP 3 508 239 B1**
(45) Veröffentlichungstag und Bekanntmachung des Hinweises auf die Patenterteilung: **23.12.2020**
(21) Anmeldenummer: 19153192.0
(22) Anmeldetag: 05.05.2010
(51) Int. Cl.: A61M 15/00, A61M 16/08, A61M 39/26, A61M 11/06, B05B 11/00

(54) **ADAPTER, INHALATIONSEINRICHTUNG UND ZERSTÄUBER**
ADAPTER, INHALANT APPARATUS AND ATOMIZER
ADAPTATEUR, DISPOSITIF D'INHALATION ET PULVÉRISATEUR

(30) Priorität: 18.05.2009 EP 09006673
(43) Veröffentlichungstag der Anmeldung: 10.07.2019
(62) Teilanmeldung aus: 10721112.0
(73) Patentinhaber: Boehringer Ingelheim International GmbH, 55216 Ingelheim am Rhein (DE)
(72) Erfinder: WACHTEL, Herbert, 55216 INGELHEIM AM RHEIN (DE)
(74) Vertreter: Simon, Elke Anna Maria

(56) Entgegenhaltungen:
- WO-A1-02/04054
- WO-A1-93/00951
- WO-A1-2004/091704
- US-A- 5 497 765
- US-A1- 2006 254 579

## Beschreibung

Die vorliegende Erfindung betrifft eine Inhalationseinrichtung gemäß dem Oberbegriff des Anspruchs 1 sowie einen Zerstäuber, insbesondere einen Inhalator, mit einer derartigen Inhalationseinrichtung.

Zerstäuber, insbesondere Inhalatoren, dienen der Versorgung eines Benutzers oder Patienten mit einem Aerosol, also einem zerstäubten Fluid, das vorzugsweise ein Arzneimittel aufweist bzw. enthält oder eine Arzneimittelzubereitung darstellt. Bei der Verabreichung ist oftmals eine sehr genaue Dosierung wünschenswert oder erforderlich. Dementsprechend ist es wichtig, daß eine von einem Zerstäuber als Aerosol ausgegebene Dosis auch möglichst vollständig inhaliert wird.

Ein Ausgangspunkt der vorliegenden Erfindung ist ein Zerstäuber, wie im Grundprinzip in der WO 91/14468 A1 und in konkreter Ausgestaltung in der WO 97/12687 A1 (Fig. 6a, 6b) dargestellt. Der bekannte Zerstäuber weist einen Druckerzeuger zur Förderung und Zerstäubung einer Arzneimittelzubereitung auf. Die Arzneimittelzubereitung wird als Aerosol über ein Mundstück ausgegeben.

Problematisch ist bei Zerstäubern generell, daß ein Auslösen der Zerstäubung und das Einatmen koordiniert werden müssen. Dies kann für den einzelnen Benutzer schwierig sein.

Die WO 2004/091704 A1 offenbart eine Inhalationseinrichtung zur Zwischenspeicherung eines erzeugten Aerosols in einer Kammer. Die bekannte Inhalationseinrichtung ist für einen MDI (Metered Dose Inhaler) vorgesehen und dient einer Verlangsamung des Aerosols, insbesondere durch Verlängerung des Strömungswegs. Aus diesem Grunde werden derartige Inhalationseinrichtungen auch Spacer genannt. Des weiteren dient die Inhalationseinrichtung einer Zwischenspeicherung des erzeugten Aerosols, damit der Benutzer genügend Zeit hat, um das Aerosol zu inhalieren.

Beatmungsgeräte und -systeme dienen der Versorgung eines Patienten mit einem Atemgas, meistens über mindestens eine gasführende Schlauchleitung. Auch bei beatmeten Patienten kann eine inhalative Behandlung vorgesehen sein, bei der ein von einem Zerstäuber erzeugtes Aerosol in das Atemgas gegeben bzw. mit dem Atemgas mit eingeatmet bzw. inhaliert wird.

Die WO 2007/141201 A1 offenbart einen Adapter mit einem ersten Anschluß für einen Zerstäuber und einem zweiten patientenseitigen Anschluß. Der bekannte Adapter weist einen dritten Anschluß für einen Atemluftschlauch zur Zuführung eines Atemgases auf. Der Adapter ist also zur Verbindung mit einer Beatmungseinrichtung bzw. einem Beatmungsschlauch ausgebildet. Die darüber zugeführte Atemluft wird zum ersten Anschluß des Adapters geführt und dort neben einer in den zweiten Anschluß des Adapters ragenden Düse des zugeordneten Zerstäubers umgelenkt und zusammen mit dem von dem Zerstäuber erzeugten Aerosol über den zweiten Anschluß ausgegeben.

Die WO 93/00951 A1 offenbart eine Vorrichtung für die Abgabe von dosiertem Medikamenten in Aerosolform zur Einatmung durch einen Patienten. Die Vorrichtung umfasst einen Dosis-Regulierer, einen Zähler, eine Uhr und einen Signalgeber zur Anzeige, wann eine Dosis zur Inhalation bereit ist. Das Aeorosol wird über ein Mundstück aus einer Kammer eingeatmet, die einen Einlass für das Aerosol und einen Lufteinlass mit einem Einwegventil aufweist.

Die US 5497765 offenbart ein Gerät für die gleichzeitige Verabreichung eines Medikaments und Sauerstoff an einen Patienten. Das Gerät weist eine Hauptkammer mit einem Adapter für den Anschluss eines MDIs an einer Seite und eine Maske oder Mundstück an der anderen Seite auf. Die Hauptkammer ist des Weiteren mit Rohr verbunden, durch das über ein Einwegventil Außenluft oder Sauerstoff in die Hauptkammer gelangen kann.

Die US 2006/0254579 A1 offenbart eine Vorrichtung, mittels der ein Medikament aus einem MDI einem Beatmungssystem zwischen einem Einlassanschluss für die Atemluftzufuhr und einem patientenseitigen Auslassanschluss zugeführt werden kann. Die Vorrichtung weist einen Innenraum in Form einer Haltekammer in fluidischer Verbindung mit dem MDI auf, wobei die Kammer eine Einlassöffnung mit Einweg-Ventil für die Zufuhr von Beatmungsluft und eine Auslassöffnung (in Richtung Patient) aufweist. Die Vorrichtung weist zwischen Einlass- und Auslassanschluss einen Bypass zur Haltekammer auf, wobei im Bypass ein Einwegventil angeordnet ist, das über den Auslassanschluss ins System kommmende Ausatemluft des Patienten durchlässt.

Der vorliegenden Erfindung liegt die Aufgabe zugrunde, einen einfachen und/oder universell einsetzbaren Adapter für einen Zerstäuber, eine verbesserte Inhalationseinrichtung mit einer Kammer zur Zwischenspeicherung eines von einem Zerstäuber erzeugten Aerosols und einen Zerstäuber mit einem derartigen Adapter und/oder einer derartigen Inhalationseinrichtung anzugeben.

Die obige Aufgabe wird durch eine Inhalationseinrichtung gemäß Anspruch 1 oder einen Zerstäuber gemäß Anspruch 11 gelöst. Vorteilhafte Weiterbildungen sind Gegenstand der Unteransprüche.

Gemäß einem ersten Aspekt der vorliegenden Erfindung ist eine Inhalationseinrichtung mit einer Kammer zur Zwischenspeicherung eines Aerosols vorgesehen, wobei die Kammer einlaßseitig mit einem ersten Anschluß für einen Zerstäuber - zumindest bei angeschlossenem Zerstäuber - ventilfrei fluidisch verbunden und parallel einlaßseitig über ein Einlaßventil an einen Anschluß zur Zuleitung von Atemluft angeschlossen ist, so daß Atemluft vom letztgenannten Anschluß über das Einlaßventil in die Kammer strömen kann. Dies gestattet insbesondere ein ungehindertes bzw. weitgehend verlustfreies Einströmen von Aerosol in die Kammer, so daß ein unerwünschtes Niederschlagen von zerstäubtem Fluid an einem einlaßseitigen Ventil in der Kammer vermieden werden kann.

Insbesondere ist die Kammer auch auslaßseitig ventilfrei an einen patientenseitigen Anschluß angeschlossen, so daß das Aerosol weitgehend ungehindert und verlustfrei bei der Entnahme aus der Kammer über den patientenseitigen Anschluß ausströmen kann.

Gemäß einem zweiten Aspekt der vorliegenden Erfindung ist eine Inhalationseinrichtung mit einer Kammer zur Zwischenspeicherung von Aerosol vorgesehen, wobei die Kammer auslaßseitig mehrere zumindest im wesentlichen ringförmig angeordnete Auslaßöffnungen aufweist. Dies gestattet überraschenderweise ein relativ verlustfreies Ausströmen des Aerosols aus der Kammer.

Gemäß einem dritten Aspekt der vorliegenden Erfindung ist ein Adapter vorgesehen, der einen ersten Anschluß mit einem ovalen Querschnitt zur Verbindung mit einem ovalen Mundstück eines Zerstäubers aufweist. Dies gestattet eine sehr einfache Verbindung mit dem zugeordneten Zerstäuber bzw. dessen Mundstück.

Gemäß einem vierten Aspekt der vorliegenden Erfindung ist ein Adapter vorgesehen, der einen ersten Anschluß mit einem Stutzen zur Aufnahme einer Düse des Zerstäubers aufweist. Dies gestattet auf sehr einfache Weise eine gute fluidische Verbindung mit dem zugeordneten Zerstäuber.

Gemäß einem fünften Aspekt der vorliegenden Erfindung ist ein Adapter vorgesehen, bei dem der erste Anschluß für den Zerstäuber und ein zweiter patientenseitiger Anschluß unverzweigt miteinander verbunden sind. Insbesondere ist der zweite Anschluß zur Verbindung mit einem Schlauch oder einer Inhalationseinrichtung ausgebildet. Dies gestattet einen besonders einfachen Aufbau und ermöglicht einen besonders universellen Einsatz des zugeordneten Zerstäubers zusammen mit dem Adapter, insbesondere zum Anschluß an Beatmungssysteme o. dgl. Aufgrund des sehr einfachen Aufbaus wird der Adapter vorzugsweise als Wegwerfartikel bzw. nur einmalig verwendet.

Gemäß einem sechsten Aspekt der vorliegenden Erfindung ist der Adapter mit einer Inhalationseinrichtung mit einer Kammer zur Zwischenspeicherung von Aerosol verbunden oder verbindbar, insbesondere lösbar. Dies gestattet bei einfachem Aufbau die Bildung eines modularen Systems, das sehr universell einsetzbar ist. Bedarfsweise kann der Adapter auch gewechselt oder nur einmalig verwendet werden, wohingegen die Inhalationseinrichtung bedarfsweise mehrfach verwendet werden kann.

Gemäß einem siebten Aspekt der vorliegenden Erfindung ist ein Zerstäuber in Kombination mit einem vorgenannten Adapter und/oder einer vorgenannten Inhalationseinrichtung vorgesehen. Dies gestattet einen besonders universellen Einsatz des Zerstäubers, insbesondere auch bei beatmeten Patienten bzw. in Kombination mit Beatmungsgeräten bzw. -systemen.

Die vorgenannten Aspekte der vorliegenden Erfindung sowie die sich aus der weiteren Beschreibung und den Ansprüchen ergebenden Merkmale und Aspekte der Erfindung können unabhängig voneinander und in beliebiger Kombination realisiert werden.

Weitere Vorteile, Merkmale, Eigenschaften und Aspekte der vorliegenden Erfindung ergeben sich aus den Ansprüchen und der folgenden Beschreibung einer bevorzugten Ausführungsform anhand der Zeichnung. Es zeigt:
- Fig. 1: eine schematische Darstellung eines Adapters gemäß eines ersten Beispiels mit einem angeschlossenen Zerstäuber;
- Fig. 2: einen schematischen Schnitt eines Zerstäubers im ungespannten Zustand;
- Fig. 3: einen schematischen, um 90° gegenüber Fig. 2 gedrehten Schnitt des Zerstäubers im gespannten Zustand;
- Fig. 4: einen schematischen Schnitt des Zerstäubers mit angeschlossenem Adapter;
- Fig. 5: einen schematischen Schnitt eines Adapters gemäß eines zweiten Beispiels;
- Fig. 6: eine perspektivische Ansicht des Adapters gemäß Fig. 5;
- Fig. 7: eine Draufsicht des Adapters gemäß Fig. 5;
- Fig. 8: eine perspektivische Ansicht eines Adapters gemäß eines dritten Beispiels mit einer angeschlossenen Inhalationseinrichtung;
- Fig. 9: eine Schnittdarstellung eines Adapters gemäß Fig. 8 bei nicht angeschlossenem Zerstäuber;
- Fig. 10: eine Schnittdarstellung eines Adapters gemäß Fig. 8 bei angeschlossenem Zerstäuber;
- Fig. 11: einen schematischen Schnitt einer vorschlagsgemäßen Inhalationseinrichtung gemäß einer Ausführungsform mit einem zugeordneten Adapter;
- Fig. 12: eine ausschnittsweise Vergrößerung von Fig. 11;
- Fig. 13: einen schematischen Schnitt der Inhalationseinrichtung beim Ausatmen;
- Fig. 14: einen schematischen Schnitt der Inhalationseinrichtung beim Einatmen; und
- Fig. 15: einen schematischen Schnitt einer vorschlagsgemäßen Inhalationseinrichtung gemäß einer zweiten Ausführungsform beim Einatmen.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

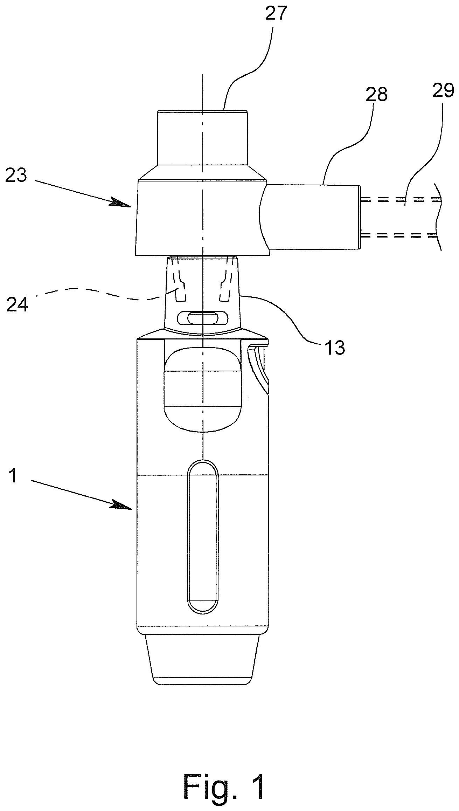

Fig. 1 zeigt in einer schematischen Darstellung eines Adapters 23 mit einem zugeordneten, in der Darstellung angeschlossenen Zerstäuber 1.

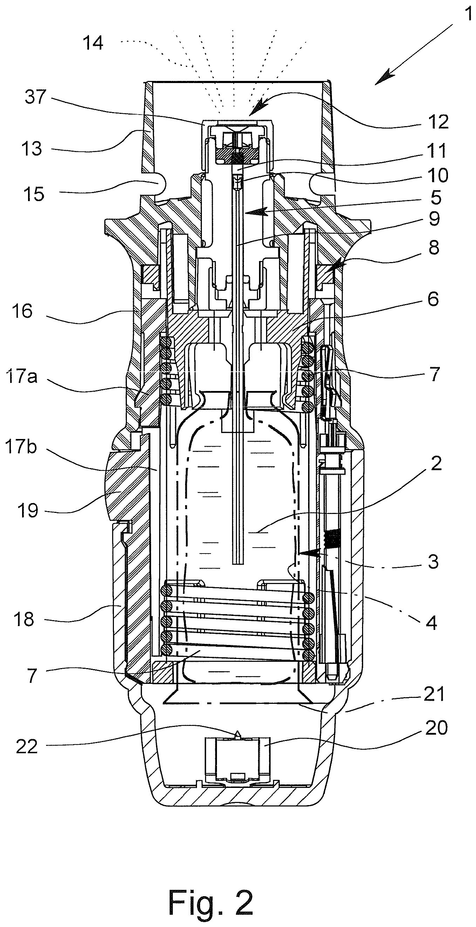

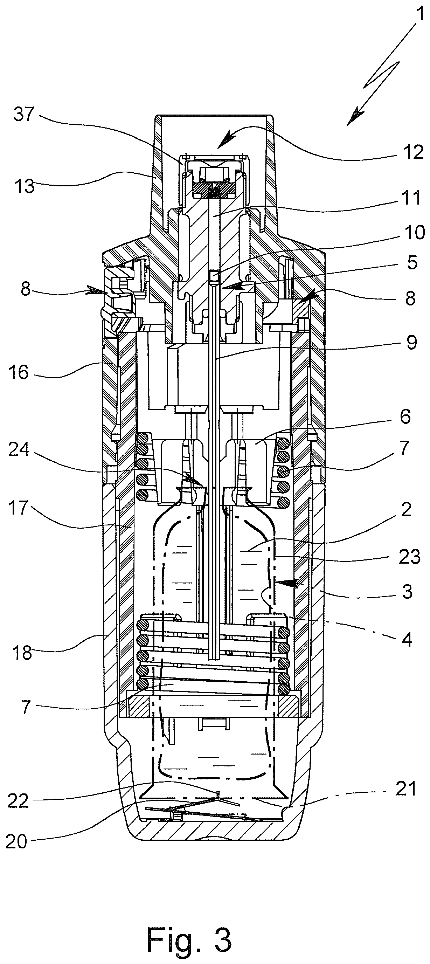

Fig. 2 und 3 zeigen den vorzugsweise tragbaren Zerstäuber 1 zur treibgasfreien Zerstäubung eines Fluids, vorzugsweise einer Flüssigkeit bzw. Arzneimittelzubereitung 2 in einer schematischen Darstellung im ungespannten Zustand (Fig. 2) und gespannten Zustand (Fig. 3). Fig. 2 und 3 zeigen den Zerstäuber 1 mit einem Behälter 3 mit der Arzneimittelzubereitung 2.

Bei Zerstäubung der Arzneimittelzubereitung 2, vorzugsweise einer Flüssigkeit, wird ein lungengängiges Aerosol 14 (Fig. 2) gebildet, das von einem nicht dargestellten Benutzer bzw. Patienten eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten.

Der Zerstäuber 1 weist den vorzugsweise einsetzbaren und ggf. wechselbaren Behälter 3 mit der Arzneimittelzubereitung 2 auf. Der Behälter 3 bildet also ein Reservoir für die zu zerstäubende Arzneimittelzubereitung 2. Vorzugsweise enthält der Behälter 3 eine ausreichende Menge an Arzneimittelzubereitung 2 bzw. Wirkstoff für mehrere Dosen der Arzneimittelzubereitung 2, um mehrere Zerstäubungen oder Anwendungen zu ermöglichen. Ein typischer Behälter 3, wie in der WO 96/06011 A1 offenbart, nimmt ein Volumen von ca. 2 bis 10 ml auf. Hinsichtlich des bevorzugten Aufbaus des Behälters 3 wird ergänzend auf die WO 00/49988 A2 verwiesen.

Der Behälter 3 ist vorzugsweise im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei die Arzneimittelzubereitung 2 in einem kollabierbaren Beutel 4 im Behälter 3 aufgenommen ist.

Der Zerstäuber 1 weist ferner eine Fördereinrichtung, insbesondere einen Druckerzeuger 5, zur Förderung und Zerstäubung der Arzneimittelzubereitung 2, insbesondere jeweils in einer vorbestimmten, ggf. einstellbaren Dosiermenge auf.

Der Zerstäuber 1 bzw. Druckerzeuger 5 weist insbesondere eine Halterung 6 für den Behälter 3, eine zugeordnete, nur teilweise dargestellte Antriebsfeder 7 vorzugsweise mit einem zugeordneten, zur Entsperrung manuell betätigbaren Sperrelement 8, ein Förderelement, vorzugsweise ein als Kapillare ausgebildetes Förderrohr 9, mit einem optionalen Ventil, insbesondere Rückschlagventil 10, eine Druckkammer 11 und/oder eine Austragsdüse 12 insbesondere im Bereich eines Mundstücks 13 auf.

Der Behälter 3 wird über die Halterung 6, insbesondere klemmend oder rastend, so in dem Zerstäuber 1 fixiert, daß das Förderrohr 9 in den Behälter 3 eintaucht. Die Halterung 6 kann dabei derart ausgebildet sein, daß der Behälter 3 ausgetauscht werden kann.

Beim axialen Spannen der Antriebsfeder 7 wird die Halterung 6 mit dem Behälter 3 und dem Förderrohr 9 bei den Darstellungen nach unten bewegt und die Arzneimittelzubereitung 2 - genauer gesagt die nächste Dosis - aus dem Behälter 3 in die Druckkammer 11 des Druckerzeugers 5 über das Rückschlagventil 10 gesaugt.

Beim anschließenden Entspannen nach Betätigung des Sperrelements 8 wird die Arzneimittelzubereitung 2 in der Druckkammer 11 unter Druck gesetzt, indem das Förderrohr 9 bei nun geschlossenem Rückschlagventil 10 durch Entspannen der Antriebsfeder 7 wieder nach oben bewegt wird und nun als Druckstempel wirkt. Dieser Druck treibt die Arzneimittelzubereitung 2 durch die Austragsdüse 12 aus, wobei es in das vorzugsweise lungengängige Aerosol 14 zerstäubt wird, wie in Fig. 2 angedeutet.

Der nicht dargestellte Benutzer bzw. Patient kann das Aerosol 14 inhalieren, wobei vorzugsweise Zuluft über mindestens eine Zuluftöffnung 15 in das Mundstück 13 saugbar ist.

Während des Zerstäubungsvorgangs wird der Behälter 3 von der Antriebsfeder 7 wieder in seine Ausgangslage zurückbewegt. Der Behälter 3 führt also eine Hubbewegung während des Spannvorgangs und während des Zerstäubungsvorgangs aus.

Der Zerstäuber 1 weist insbesondere ein erstes Gehäuseteil (Oberteil) 16 und ein demgegenüber drehbares Innenteil 17 (Fig. 3) mit einem oberen Teil 17a und einem unteren Teil 17b (Fig. 2) auf, wobei an dem Innenteil 17 ein insbesondere manuell betätigbares oder drehbares, zweites Gehäuseteil (Unterteil) 18 vorzugsweise mittels eines Sicherheitsverschlusses bzw. Halteelements 19 lösbar befestigt, insbesondere aufgesteckt, ist. Insbesondere ist der Sicherheitsverschluß bzw. das Halteelement 19 derart ausgebildet, daß ein versehentliches Öffnen des Zerstäubers 1 bzw. Abziehen des zweiten Gehäuseteils 18 ausgeschlossen ist. Insbesondere muß zum Lösen des zweiten Gehäuseteils 18 das Halteelement 19 gegen Federkraft eingedrückt werden. Zum Einsetzen und/oder Auswechseln des Behälters 3 ist das zweite Gehäuseteil 18 vom Zerstäuber 1 lösbar. Das zweite Gehäuseteil 18 bildet vorzugsweise ein kappenartiges Gehäuseunterteil und/oder um- bzw. übergreift einen unteren freien Endbereich des Behälters 3.

Das zweite Gehäuseteil 18 kann relativ zum ersten Gehäuseteil 16 gedreht werden, wobei das Innenteil 17 mitgedreht wird. Dadurch wird die Antriebsfeder 7 über ein nicht im einzelnen dargestelltes, auf die Halterung 6 wirkendes Getriebe in axialer Richtung gespannt. Mit dem Spannen wird der Behälter 3 axial nach unten bzw. mit seinem Endbereich (weiter) in das zweite Gehäuseteil 18 bzw. zu dessen stirnseitigem Ende hin bewegt, bis der Behälter 3 eine in Fig. 3 angedeutete Endlage einnimmt. In diesem Zustand ist die Antriebsfeder 7 bzw. der Zerstäuber 1 gespannt und gesperrt.

Der Zerstäuber 1 weist vorzugsweise eine Einrichtung zur zwangsweisen Belüftung des Behälters 3 auf.

Beim erstmaligen Spannen erfolgt vorzugsweise ein bodenseitiges Anstechen bzw. Öffnen des Behälters 3. Insbesondere kommt eine axial wirkende, im Gehäuseteil 18 angeordnete Feder 20 am Behälterboden 21 zur Anlage, die mit einem Anstechelement 22 den Behälter 3 bzw. eine bodenseitige, insbesondere gasdichte Versiegelung bei der erstmaligen Anlage zur Belüftung ansticht.

Die Einrichtung zur zwangsweisen Belüftung ist hier also durch das Anstechelement 22 gebildet, das von der Feder 20 gehalten oder gebildet ist. Jedoch sind auch andere konstruktive Lösungen möglich.

Es ist anzumerken, daß bei dem Anstechen zur Belüftung lediglich die Außenhülle des Behälters 3 geöffnet wird. Der Beutel 4 mit der Arzneimittelzubereitung 2 bleibt unbeschädigt. Bei der Entnahme der Arzneimittelformulierung 2 aus dem Beutel 4 über das Förderrohr 9 kollabiert der flexible Beutel 4. Zum Druckausgleich kann die Umgebungsluft über die Belüftungs- bzw. Anstechöffnung in den Behälter 3 nachströmen.

Zur Benutzung des Zerstäubers 1 muß zunächst der Behälter 3 eingesetzt werden. Dies erfolgt vorzugsweise dadurch, daß das zweite Gehäuseteil 18 entfernt bzw. abgezogen wird. Anschließend wird der Behälter 3 in das Innenteil 17 axial eingeführt bzw. eingeschoben. Hierbei erfolgt ein kopfseitiges Öffnen bzw. Anschließen des Behälters 3. Dies erfolgt durch das Förderelement, also das Förderrohr 9, das eine vorzugsweise vorgesehene, kopfseitige Versiegelung des Behälters 3 durchsticht und anschließend durch ein kopfseitiges Septum des Behälters 3 hindurch in das Innere des Beutels 4 eingeführt wird. So wird die fluidische Verbindung zwischen dem Behälter 3 - genauer gesagt zwischen dem Beutel 4 im Behälter 3 - über das Förderrohr 9 zum Druckerzeuger 5 bzw. zur Druckkammer 11 hergestellt.

Anschließend wird das zweite Gehäuseteil 18 wieder aufgeschoben. Nun kann das erstmalige Spannen des Zerstäubers 1 erfolgen. Hierbei wird dann der Behälter 3 durch das Anstechelement 22 bodenseitig angestochen, also zwangsweise belüftet, wie bereits erläutert.

Vor der erstmaligen Benutzung erfolgt nach dem Einsetzen bzw. fluidischen Anschließen des Behälters 3 ein vorzugsweise mehrmaliges Spannen und Auslösen des Zerstäubers 1. Durch dieses sogenannte Primen wird im Förderrohr 9 und im Druckerzeuger 5 bis zur Austragsdüse 12 eventuell vorhandene Luft von der Arzneimittelzubereitung 2 verdrängt. Anschließend ist der Zerstäuber 1 zur Inhalation bereit.

Die Menge der pro Hub bzw. Zerstäubungsvorgang ausgebrachten Arzneimittelzubereitung 2 beträgt vorzugsweise etwa 10 µl bis 50 µl, insbesondere etwa 10 µl bis 20 µl, ganz besonders bevorzugt etwa 15 µl.

Die Antriebsfeder 7 ist vorzugsweise vorgespannt eingebaut, um einen hohen Förderdruck zu erreichen. Beim vorschlagsgemäßen Zerstäuber 1 erfolgt das Unterdrucksetzen und Fördern der Arzneimittelzubereitung 2 während des Zerstäubungsvorgangs nämlich vorzugsweise nur durch Federkraft, insbesondere nur durch die Kraft der Antriebsfeder 7.

Der Zerstäuber 1 ist vorzugsweise derart ausgebildet, daß die Arzneimittelzubereitung 2 im Druckerzeuger 5 bzw. in der Druckkammer 11 bei der Ausgabe einem Druck von 5 MPa bis 60 MPa, insbesondere etwa 10 MPa bis 50 MPa, erreicht. Besonders bevorzugt wird bei der Ausgabe bzw. Zerstäubung der Arzneimittelzubereitung 2 ein Druck von etwa 5 MPa bis 60 MPa, insbesondere etwa 10 bis 30 MPa, an der Austragsdüse 12 bzw. an deren Düsenöffnungen erreicht. Die Arzneimittelzubereitung 2 wird dann in das Aerosol 14 überführt, dessen Tröpfchen einen aerodynamischen Durchmesser von bis zu 20 µm, bevorzugt etwa 3 µm bis 10 µm, aufweisen. Die Zerstäubungswirkung bzw. der Zerstäubungseffekt wird durch sich vorzugsweise kreuzende Strahlen, die von der Austragsdüse 12 abgegeben werden, bewirkt bzw. weiter unterstützt.

Der Zerstäuber 1 ist vorzugsweise derart ausgebildet, daß das Aerosol 14 mit geringer Geschwindigkeit, insbesondere mit einer Geschwindigkeit von weniger als 2 m/s, besonders bevorzugt von etwa 1,6 m/s oder weniger, ausgegeben wird (jeweils gemessen in 10 cm Abstand von der Austragsdüse 12). Der Zerstäuber 1 ist also vorzugsweise als sogenannter Soft Mist Inhaler ausgebildet. Die geringe Ausgabegeschwindigkeit kann insbesondere durch sich kreuzende Strahlen der Arzneimittelzubereitung 2, die von der Austragsdüse 12 abgegeben werden, und/oder entsprechende Wahl der Federkraft bewirkt bzw. unterstützt werden.

Besonders bevorzugt ist der Zerstäuber 1 derart ausgebildet, daß die Aerosolerzeugung jeweils über mindestens 1 s, insbesondere über mindestens 1,5 s, dauert. Die Zeitdauer zur Zerstäubung einer Dosis bzw. bei einer Betätigung des Zerstäubers 1 beträgt also mindestens 1 s, insbesondere über 1,5 s.

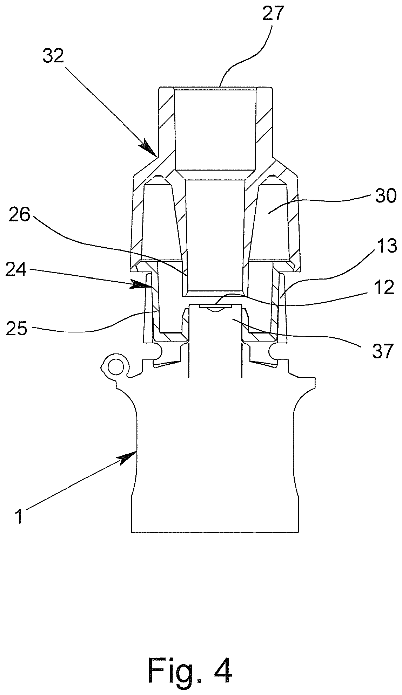

Fig. 4 zeigt in einem schematischen Schnitt den Adapter 23 und nur noch schematisch angedeutet Teile des angeschlossenen Zerstäubers 1.

Der Adapter 23 ist vorzugsweise lösbar und/oder insbesondere klemmend oder rastend mit dem Zerstäuber 1 bzw. dessen Mundstück 13 verbunden oder verbindbar.

Der Adapter 23 weist einen ersten Anschluß 24 für den Zerstäuber 1, genauer gesagt zur fluidischen und vorzugsweise auch mechanischen Verbindung mit dem Zerstäuber 1 bzw. dessen Mundstück 13, auf.

Der erste Anschluß 24 weist beim Darstellungsbeispiel vorzugsweise einen Anschlußabschnitt 25 auf, der sich in das Mundstück 13 erstreckt, insbesondere in dieses einsteckbar ist. Der Anschlußabschnitt 25 ist dementsprechend in seiner Außenkontur an die Innenkontur des Mundstücks 13 angepaßt. Beispielsweise verjüngt sich der Anschlußabschnitt 25 auf seiner Außenseite zum freien Ende hin und ist damit zumindest im wesentlichen komplementär zu einer vorzugsweise leicht konischen Form des Mundstücks 13 ausgebildet. Jedoch sind auch andere konstruktive Lösungen möglich.

Beim Darstellungsbeispiel weist der erste Anschluß 24 bzw. dessen Anschlußabschnitt 25 vorzugsweise einen ovalen Querschnitt zur Verbindung mit dem vorzugsweise ovalen Mundstück 13 des Zerstäubers 1 auf.

Beim Darstellungsbeispiel verschließt der Anschlußabschnitt 25 vorzugsweise a) den im wesentlichen ringförmigen Zwischenraum zwischen der in das Mundstück 13 mit einer Halterung oder einem Vorsprung 37 vorragenden Austragsdüse 12 und der Innenwand des Mundstücks 13 und/oder b) unmittelbar die Zuluftöffnungen 15, insbesondere jeweils so, daß Zuluft über die Zuluftöffnung(en) 15 nur vermindert oder gar nicht mehr in das Mundstück 13 bei der Benutzung des Zerstäubers 1 bzw. Adapters 23 einströmt.

Der Anschluß 24 weist vorzugsweise einen Stutzen 26 oder sonstige insbesondere kanalartigen Abschnitt auf, der bei angeschlossenem Zerstäuber 1 der Austragsdüse 12 zugeordnet ist, insbesondere diese abdeckt oder aufnimmt bzw. benachbart zu dieser angeordnet ist, um das vom Zerstäuber 1 bzw. der Austragsdüse 12 abgegebene Aerosol 14 aufzunehmen bzw. weiterzuleiten.

Beim Darstellungsbeispiel endet der Stutzen 26 axial beabstandet zu der Austragsdüse 12 bzw. einer der Austragsdüse 12 zugeordneten Halterung, so daß Zuluft oder Atemluft einer Beatmungseinrichtung (seitlich) zusammen mit dem Aerosol 14 in den Stutzen 26 einströmen kann bzw. an der Austragsdüse 12 vorbeiströmen kann.

Jedoch ist es grundsätzlich auch möglich, daß der erste Anschluß 24 bzw. Stutzen 26 (zumindest im wesentlichen) dicht mit der Austragsdüse 12 bzw. einem die Austragsdüse 12 haltenden oder umgebenden Vorsprung 37 verbindbar ist, besonders bevorzugt durch Aufstecken des Stutzens 26, so daß keine Zuluft oder Atemluft an der Austragsdüse 12 vorbei durch oder in den ersten Anschluß 24 einströmen kann.

Der Adapter 23 weist einen zweiten patientenseitigen Anschluß 27 auf. Der zweite Anschluß 27 ist vorzugsweise rohrförmig oder als Bohrung ausgebildet und/oder weist insbesondere einen zumindest im wesentlichen runden Querschnitt auf.

Der zweite Anschluß 27 ist vorzugsweise zur mechanischen und/oder fluidischen Verbindung mit einem Schlauch, einer Beatmungseinrichtung oder einer Inhalationseinrichtung ausgebildet. Jedoch kann der zweite Anschluß 27 auch grundsätzlich als Mundstück ausgebildet sein.

Beim Darstellungsbeispiel weist der Adapter 23 vorzugsweise einen nur in Fig. 1 dargestellten dritten Anschluß 28 für Zu- oder Atemluft auf. Der dritte Anschluß 28 ist vorzugsweise zur mechanischen und/oder fluidischen Verbindung mit einem nur gestrichelt angedeuteten Beatmungsschlauch 29 oder einer sonstigen Beatmungseinrichtung o. dgl. ausgebildet. Hierzu ist der dritte Anschluß 28 vorzugsweise stutzen-, rohr- oder tubusförmig ausgebildet und/oder so, daß der Schlauch 29 o. dgl. vorzugsweise einsteckbar oder aufsteckbar ist.

Die vorliegende Erfindung bezieht sich vorzugsweise auf die Verwendung bei einem beatmeten Patienten bzw. mit einer Beatmungseinrichtung. Dementsprechend wird im folgenden meistens von Atemluft gesprochen. Der Begriff "Atemluft" ist dann vorzugsweise als ein Beatmungsgas zu verstehen, das von einer Beatmungseinrichtung bzw. einem Beatmungssystem zur Beatmung eines Patienten bereitgestellt wird. Grundsätzlich kann es sich bei der Atemluft jedoch auch um sonstige Zuluft und/oder auch um Ausatemluft, insbesondere bei umgekehrter Strömungsrichtung handeln. Der Begriff "Atemluft" ist dementsprechend vorzugsweise sehr breit zu verstehen, so daß diese Alternativen vorzugsweise mit abgedeckt sind.

Der dritte Anschluß 28 ist vorzugsweise von dem Adapter 23 gebildet bzw. an diesen angeformt.

Über den dritten Anschluß 28 ist Atemluft vorzugsweise über einen vom oder im Adapter 23 gebildeten Ringkanal 30 dem ersten Anschluß 24 oder zweiten Anschluß 27 zuführbar, so daß diese mit dem Aerosol 14 mischbar ist und/oder zusammen mit dem Aerosol 14 über den zweiten Anschluß 27 ausgebbar ist.

Beim Darstellungsbeispiel bildet die Atemluft vorzugsweise einen Hüllstrom für das von der Austragsdüse 12 abgebende Aerosol 14. Dies wird hier vorzugsweise dadurch erreicht, daß die Atemluft zumindest im wesentlichen ringförmig und/oder mit einem Drall im Bereich der Austragsdüse 12 geführt wird und dann durch den ersten Anschluß 24 bzw. Stutzen 26 zusammen mit dem Aerosol 14 (das in Fig. 4 nicht dargestellt ist) zu dem zweiten Anschluß 27 strömen kann. Im Adapter 23 ist hierzu eine entsprechende fluidische Verbindung gebildet.

Das Aerosol 14 und die Atemluft können dann über den zweiten Anschluß 27 an den nicht dargestellten Patienten, beispielsweise über einen daran angeschlossenen Beatmungsschlauch, über eine Gesichtsmaske o. dgl. ausgegeben werden.

Es ist anzumerken, daß der dritte Anschluß 28 nur optional ist. Anstelle des dritten Anschlusses 28 kann, sofern erforderlich, Zuluft bzw. Atemluft über die Zuluftöffnung(en) 15 des Zerstäubers 1 o. dgl. alternativ zugeführt werden.

Nachfolgend werden weitere bevorzugte Ausführungsformen erläutert. Die bisherigen Ausführungen und Erläuterungen gelten insbesondere ergänzend, auch wenn eine wiederholte Beschreibung weggelassen ist.

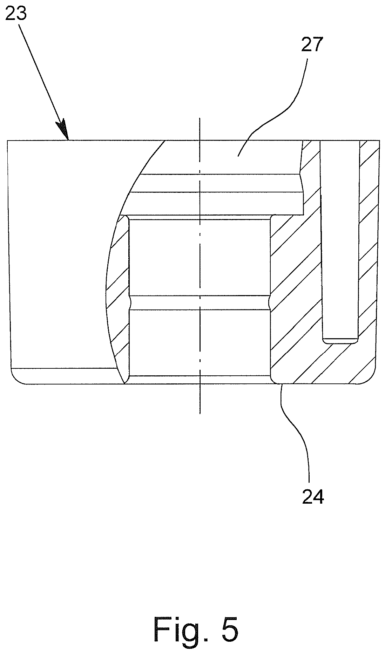

Fig. 5 zeigt in einem schematischen Schnitt eines zweiten Beispiels des Adapters 23.

Der Adapter 23 ist vorzugsweise als Wegwerfartikel ausgebildet bzw. nur einmalig verwendbar. Dies ist aufgrund des besonders einfachen Aufbau des Adapters 23 gemäß der zweiten Ausführungsform auch ökonomisch sinnvoll, da eine Reinigung und insbesondere Sterilisierung des Adapters 23 nach einem Gebrauch dann nicht mehr erforderlich ist.

Bei dem zweiten Beispiel ist der erste Anschluß 24 fluidisch unverzweigt und insbesondere einstückig mit dem zweiten Anschluß 27 verbunden. Insbesondere weist der Adapter 23 keinen dritten Anschluß 28 auf. Eine Zuführung von Atemluft oder Zuluft unterbleibt also zumindest adapterseitig.

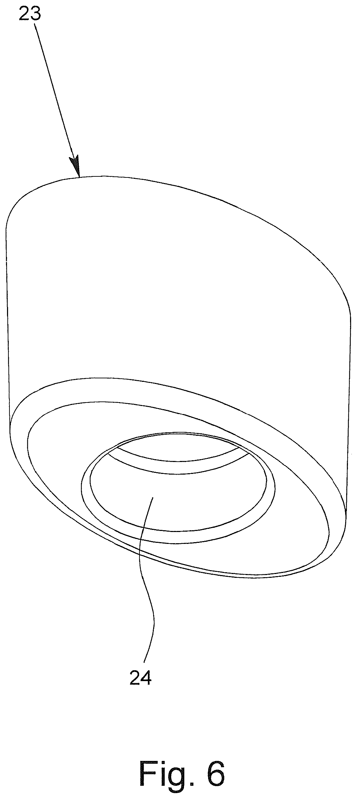

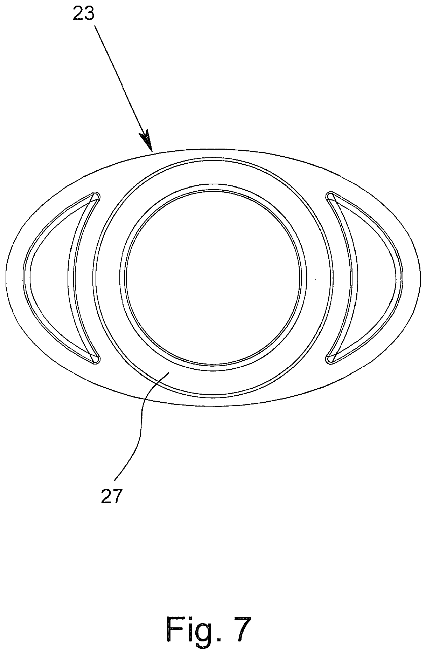

Vorzugsweise weist der erste Anschluß 24 bzw. der Adapter 23 selbst einen ovalen Querschnitt, hier eine ovale Außenkontur, zur Verbindung mit dem vorzugsweise ovalen Mundstück 13 des Zerstäubers 1, hier durch Einstecken in das Mundstück 13, auf. Die bevorzugte ovale Form ist aus der perspektivischen Darstellung gemäß Fig. 6 und der Draufsicht gemäß Fig. 7 ersichtlich.

Der Adapter 23 ist vorzugsweise einstückig ausgebildet.

Vorzugsweise ist der Adapter 23 als Spritzgußteil ausgebildet und/oder aus Kunststoff hergestellt.

Bei dem zweiten Beispiel sind die beiden Anschlüsse 24, 27 zumindest im wesentlichen durch eine vorzugsweise gerade Bohrung miteinander verbunden. Der erste Anschluß 24 weist eine vorzugsweise hohlzylindrische Innenkontur zur Aufnahme der Austragsdüse 12 bzw. des davon abgegebenen Aerosols 14 auf. Besonders bevorzugt ist ein die Austragsdüse 12 haltender oder umgebender, insbesondere zylindrischer Vorsprung 37 des Zerstäubersin den ersten Anschluß 24 einsteckbar. So kann der Austragsdüse 12 im wesentlichen gasdicht an den ersten Anschluß 24 angeschlossen werden. In diesem Fall kann keine Zuluft über die Zuluftöffnungen 15 in den ersten Anschluß 24 zuströmen.

Jedoch ist es grundsätzlich auch möglich, daß Zuluft über mindestens eine Zuluftöffnung 15 des Zerstäubers 1 o. dgl. mit in den ersten Anschluß 24 und zusammen mit dem Aerosol 14 zum zweiten Anschluß 27 strömt.

Der zweite (patientenseitige) Anschluß 27 ist vorzugsweise buchsenartig und/oder hinterschnitten ausgebildet, so daß er insbesondere rastend und/oder klemmend mit einem Schlauch, einer Inhalationseinrichtung, einem Mundstück, einer Gesichtsmaske o. dgl. verbindbar ist. Jedoch sind hier auch andere konstruktive Lösungen möglich.

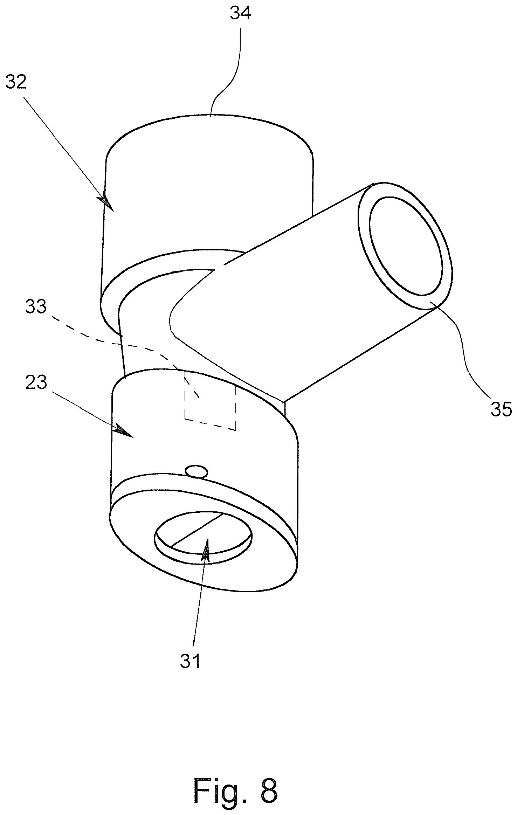

Fig. 8 zeigt in einer perspektivischen Darstellung den Adapter 23 gemäß einem dritten Beispiel.

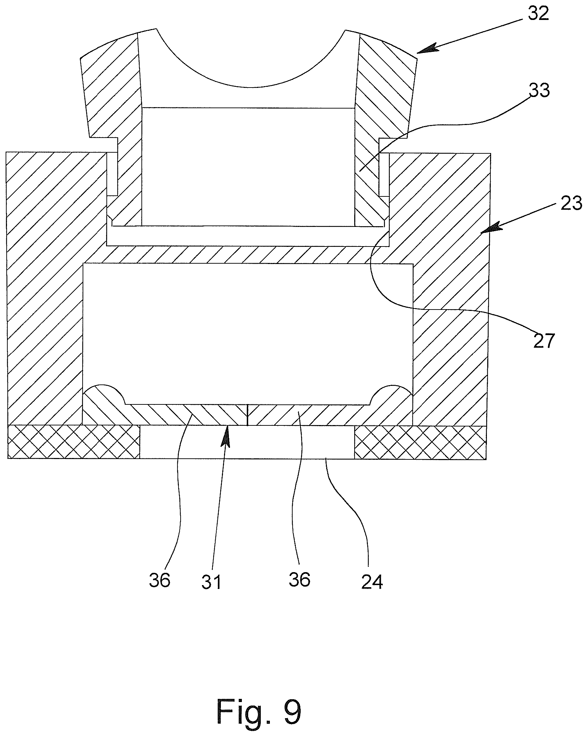

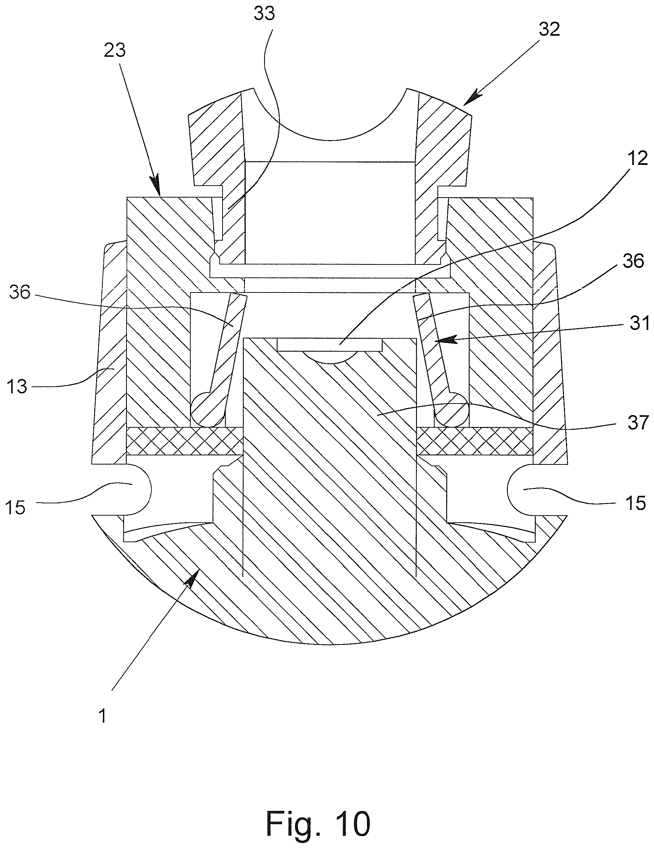

Bei dem dritten Beispiel weist der Adapter 23 ein Sperrventil 31 auf, das bei mit dem Zerstäuber 1 verbundenem Adapter 23 zwangsweise geöffnet und bei vom Zerstäuber 1 getrenntem Adapter 23 geschlossen ist. Fig. 9 zeigt in einem schematischen Schnitt den Adapter 23 bei geschlossenem Sperrventil 31, also im Zerstäuber 1 getrennten Zustand. Fig. 10 zeigt in einem schematischen Schnitt den Adapter 23 mit geöffnetem Sperrventil 31, nämlich im an den Zerstäuber 1 angeschlossenen Zustand.

Fig. 8 zeigt den Adapter 23 mit einer auslaßseitig, also an den zweiten Anschluß 27 angeschlossenen Inhalationseinrichtung 32, die hier insbesondere ein Winkelstück bildet.

Fig. 9 zeigt in dem schematischen Schnitt einen Teil der Inhalationseinrichtung 32, und zwar einen ersten Anschluss 33 für den Zerstäuber 1, der hier mittelbar über den Adapter 23 anschließbar ist. Dementsprechend ist der Adapter 23 mit seinem zweiten Anschluß 27 an den ersten Anschluß 33 der Inhalationseinrichtung 32 angeschlossen bzw. anschließbar, insbesondere mechanisch und/oder fluidisch. Beim Darstellungsbeispiel sind die beiden Anschlüsse 27 und 33 insbesondere klemmend oder rastend miteinander verbunden, hier durch Einstecken des Anschlusses 33 in den Anschluß 27.

Beim Darstellungsbeispiel erfolgt die Verbindung der Inhalationseinrichtung 32 mit dem Zerstäuber 1 nicht unmittelbar, sondern mittelbar über den Adapter 23. Dies stellt eine bevorzugte Ausführungsvariante dar. Insbesondere kann der Adapter 23 auswechselbar sein, besonders bevorzugt wenn er lösbar mit der Inhalationseinrichtung 32 verbunden oder verbindbar ist.

Die Inhalationseinrichtung 32 weist vorzugsweise einen zweiten, patientenseitigen Anschluß 34 zur Abgabe von Atemluft und von über den Adapter 23 vorzugsweise zugemischtem Aerosol 14 (nicht dargestellt) auf.

Die Inhalationseinrichtung 32 weist vorzugsweise einen dritten Anschluß 35 zur Zuleitung von Atemluft, insbesondere zur Verbindung mit einem hier nicht dargestellten Schlauch, einer nicht dargestellten Beatmungseinrichtung o. dgl. auf.

In dem schematischen Schnitt gemäß Fig. 10 ist zusätzlich ein Teil des angeschlossenen Zerstäubers 1 schematisch dargestellt. Beim Darstellungsbeispiel weist das Sperrventil 31 mindestens ein, hier zwei bewegbare Ventilelemente 36 auf, die insbesondere durch die Austragsdüse 12 bzw. den die Austragsdüse 12 haltenden Vorsprung 37 des Zerstäubers 1 geöffnet werden. Jedoch sind hier auch andere konstruktive Lösungen möglich.

Das Sperrventil 31 ist vorzugsweise in die Schließstellung vorgespannt und/oder selbst schließend ausgebildet, insbesondere mittels mindestens einem nicht dargestellten Rückstellmittel, wie einer Feder o.dgl.

Bei Benutzung des Zerstäubers 1 wird das vom Zerstäuber 1 erzeugte Aerosol 14 über den Adapter 23 abgegeben, hier an die Inhalationseinrichtung 32. Von dort kann das Aerosol 14 insbesondere zusammen mit Atemluft einem nicht dargestellten Patienten zugeführt werden, der insbesondere beatmet wird. Die Beatmung erfolgt insbesondere durch entsprechende Zuführung der Atemluft.

Nachfolgend werden Ausführungsformen der erfindungsgemäßen Inhalationseinrichtung 32 erläutert. Die bisherigen Ausführungen und Erläuterungen gelten insbesondere ergänzend, auch wenn eine wiederholte Beschreibung weggelassen ist.

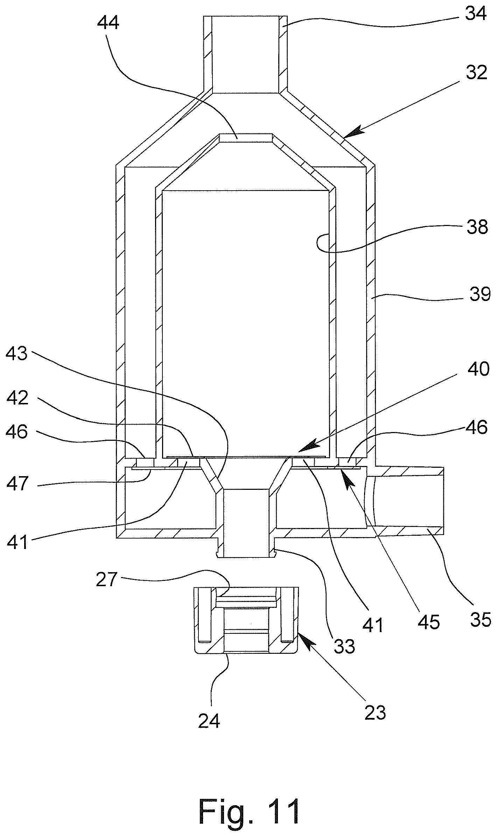

Fig. 11 zeigt in einer schematischen Schnittdarstellung die vorschlagsgemäße Inhalationseinrichtung 32 gemäß einer ersten Ausführungsform.

Wie beim voranstehend beschriebenen Beispiel kann die Inhalationseinrichtung 32 über den Adapter 23 an den Zerstäuber 1 angeschlossen werden. Jedoch kann der erste Anschluß 33 der Inhalationseinrichtung 32 auch zum direkten Anschluß an den Zerstäuber 1 bzw. das Mundstück 13 ausgebildet sein. Gegebenenfalls kann der Adapter 23 oder dessen optionales Sperrventil 31 auch in die Inhalationseinrichtung 32 bzw. dessen Anschluß 33 integriert sein. Insbesondere ist dann der erste Anschluß 33 der Inhalationseinrichtung 32 zur fluidischen und/oder mechanischen Verbindung mit dem Zerstäuber 1 bzw. dessen Mundstücks 13 ausgebildet.

Die Inhalationseinrichtung 32 weist eine Kammer 38 zur Zwischenspeicherung des von dem Zerstäuber 1 erzeugten, hier nicht dargestellten Aerosols 14 auf. Insbesondere handelt es sich hier um eine Innenkammer, die innerhalb eines Gehäuses 39 der Inhalationseinrichtung 32 angeordnet ist.

Die Kammer 38 ist fluidisch vorzugsweise direkt, insbesondere ventilfrei mit dem ersten Anschluß 33 verbunden, insbesondere so, daß zumindest bei angeschlossenem Zerstäuber 1 das von dem Zerstäuber 1 erzeugte Aerosol 14 ventilfrei bzw. möglichst verlustfrei in die Kammer 38 strömen kann. Sofern das Sperrventil 31 vorgesehen ist, ist dieses beim angeschlossenen Zerstäuber 1 geöffnet, stellt also insbesondere kein von dem Aerosol 14 zu öffnendes Ventil bzw. zu überwindendes Hindernis dar.

Unter dem Begriff "verlustfrei" ist bei der vorliegenden Erfindung insbesondere zu verstehen, daß ein unerwünschtes Niederschlagen des Aerosols 14 bzw. des zerstäubten Fluids weitgehend verhindert oder zumindest minimiert wird.

Die Kammer 38 ist einlaßseitig nicht nur mit dem ersten Anschluß 33, sondern vorzugsweise parallel einlaßseitig über ein Einlaßventil 40 an den dritten Anschluß 35 der Inhalationseinrichtung 32 angeschlossen, so daß Atemluft vom dritten Anschluß 35 in die Kammer 38 bzw. durch die Kammer 38 strömen kann, insbesondere zumindest im wesentlichen parallel zu der Hauptströmungsrichtung des Aerosols 14.

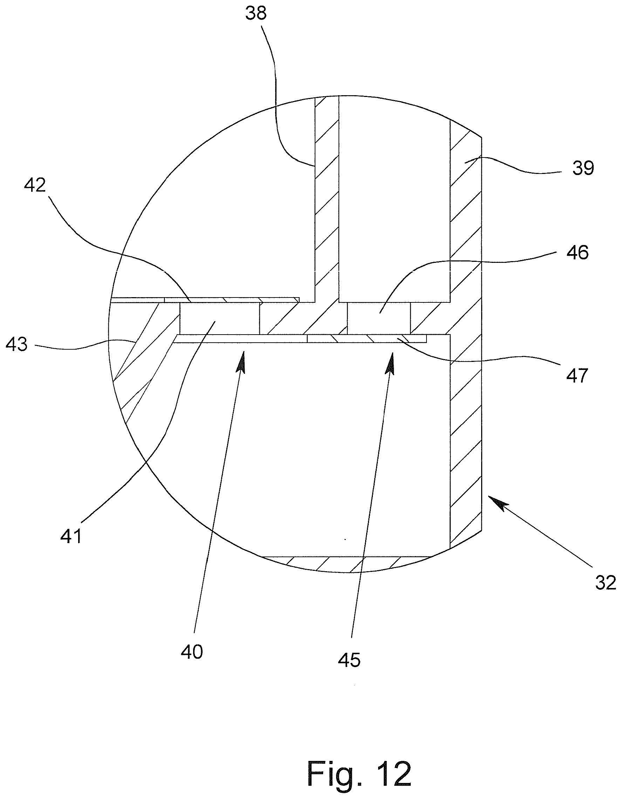

Das Einlaßventil 40 ist in Fig. 11 und in der ausschnittsweisen Vergrößerung von Fig. 11 gemäß Fig. 12 schematisch angedeutet. Insbesondere handelt es sich hier um ein Einwege- bzw. Rückschlagventil. Das Einlaßventil 40 ist vorzugsweise selbstschließend ausgebildet und/oder in die Schließstellung (leicht) vorgespannt.

Beim Darstellungsbeispiel weist das Einlaßventil 40 vorzugsweise mehrere Einlaßöffnungen 41 auf, die insbesondere von einem gemeinsamen Ventilelement 42 oder mehreren separaten Ventilelementen 42 abdeckbar bzw. verschließbar sind.

Die Einlaßöffnungen 41 sind vorzugsweise um einen zentralen oder mittigen Verbindungskanal 43 herum angeordnet, der den ersten Einlaß 33 mit der Kammer 38 verbindet. Jedoch sind auch andere konstruktive Lösungen bzw. Anordnungen möglich.

Auslaßseitig ist die Kammer 38 vorzugsweise ventilfrei fluidisch mit dem zweiten Anschluß 34 der Inhalationseinrichtung 32 verbunden.

Beim Darstellungsbeispiel weist die Kammer 38 eine vorzugsweise zentrale Auslaßöffnung 44 auf, an die sich dann der zweite Anschluß 34 - vorzugsweise in gerader Verlängerung der vorzugsweise zumindest im wesentlichen geraden Hauptströmungsrichtung des Aerosols 14 - anschließt. Jedoch sind auch andere konstruktive Lösungen möglich.

Die Inhalationseinrichtung 32 weist ein Auslaßventil 45 auf, über das der zweite Anschluß 34 parallel zur Kammer 38 an den dritten Anschluß 35 derart angeschlossen ist, daß Atemluft vom zweiten Anschluß 34 an der Kammer 38 vorbei über das Auslaßventil 45 zum dritten Anschluß 35 strömen kann. Zwischen der Kammer 38 bzw. einer die Kammer 38 bildenden, vorzugsweise im wesentlichen zylindrischen Wandung einerseits und dem Gehäuse 39 andererseits ist ein Strömungsweg - beim Darstellungsbeispiel ein Zwischen- oder Ringraum - gebildet, durch den die Atemluft vom zweiten Anschluß 34 über das Auslaßventil 45 zum dritten Anschluß 35 strömen kann.

Das Auslaßventil 45 weist vorzugsweise mehrere, insbesondere ringförmig über einen Umfang des Zwischenraums bzw. Ringraums verteilt angeordnete Ventilöffnungen 46 auf, die beim Darstellungsbeispiel durch ein gemeinsames oder mehrere separate Ventilelemente 47 verschließbar sind.

Das Auslaßventil 45 ist vorzugsweise als Einwege- oder Rückschlagventil ausgebildet.

Das Auslaßventil 45 bzw. dessen Ventilelement 47 ist vorzugsweise selbstschließend ausgebildet und/oder (leicht) in die Schließstellung vorgespannt.

Das Ventilelement 42 und/oder 47 ist vorzugsweise einstückig ausgebildet und/oder durch elastische Verformung aus der Schließstellung in eine Öffnungsstellung verformbar. Jedoch sind auch andere konstruktive Lösungen möglich.

Das Auslaßventil 45 ist vorzugsweise konzentrisch zum Einlaßventil 40 und/oder benachbart zum Einlaßventil 40 und/oder um das Einlaßventil 40 herum angeordnet. Jedoch sind auch andere konstruktive Lösungen oder Anordnungen möglich.

Das Einlaßventil 40 und/oder Auslaßventil 45 ist vorzugsweise im Bereich des Einlasses der Kammer 38 bzw. benachbart zum Verbindungskanal 43 angeordnet.

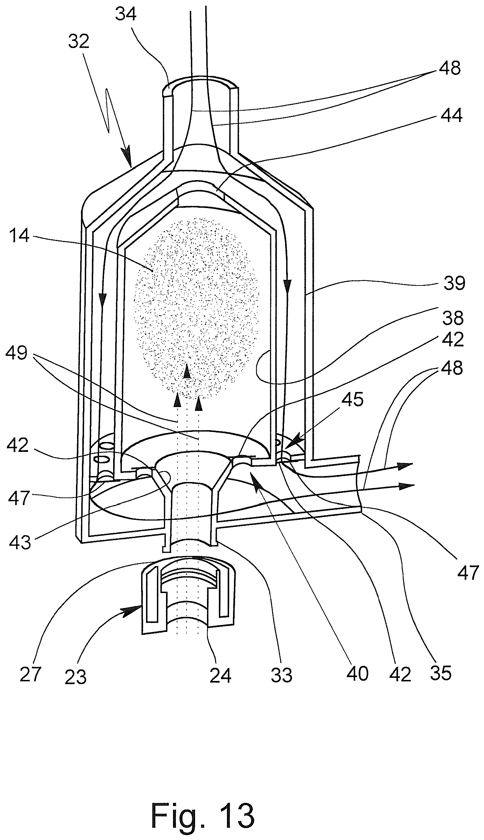

Fig. 13 veranschaulicht in einer schematischen, schnittartigen Darstellung die Luftströmung beim Ausatmen. Die Atemluft kann über den zweiten Anschluß 34 in die Inhalationseinrichtung 32 einströmen und von dort an der Kammer 38 vorbei bzw. um die Kammer 38 herum durch das sich hierbei selbsttätig öffnende Auslaßventil 45 hindurch zum dritten Anschluß 35 und aus diesem aus der Inhalationseinrichtung 32 herausströmen, wie durch Peile 48 angedeutet. Diese Ausatemluft 48 kann nicht in die Kammer 38 einströmen, da das Einlaßventil 40 in dieser Richtung geschlossen ist bzw. sich selbsttätig schließt.

In Fig. 13 ist schematisch durch gestrichelte Pfeile 49 angedeutet, wie - ggf. auch gleichzeitig oder parallel oder unabhängig davon - das Aerosol 14 vom Zerstäuber 1 (nicht dargestellt) über den optionalen Adapter 23, den ersten Anschluß 33 und den Verbindungskanal 43 in die Kammer 38 strömen kann und dort eine Wolke des Aerosols 14 bilden kann. Diese Aerosolwolke wird durch die Ausatemluft 48 nicht beeinträchtigt.

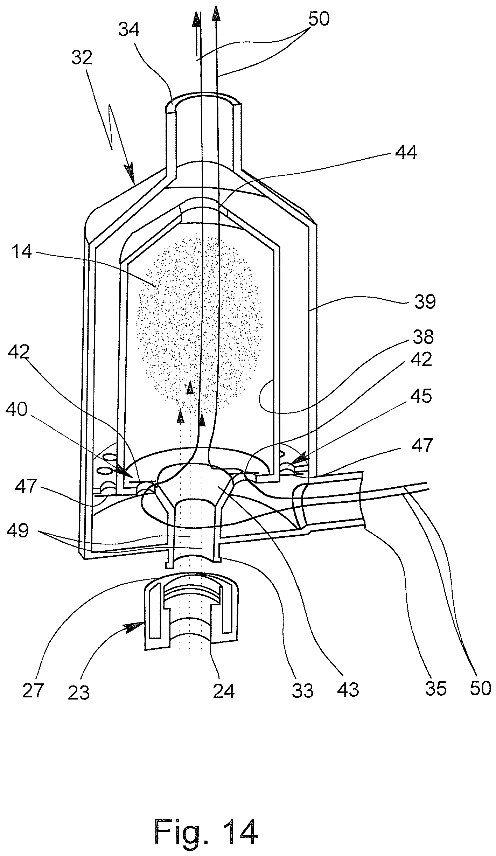

Fig. 14 zeigt die Situation beim Einatmen, also wenn Atemluft über den dritten Anschluß 35 in die Inhalationseinrichtung 32 einströmt und über den zweiten Anschluß 34 an den nicht dargestellten Patienten - insbesondere über eine nicht dargestellte Schlauchleitung, Gesichtsmaske, sonstige Beatmungseinrichtung oder ein Mundstück o. dgl. - an den nicht dargestellten Patienten abgegeben wird, wie durch Pfeile 50 angedeutet. Die Einatemluft 50 strömt durch das sich in dieser Richtung selbsttätig öffnende Einlaßventil 40 einlaßseitig in die Kammer 38 und strömt zusammen mit dem Aerosol 14, das insbesondere von der Einatemluft 50 mitgenommen wird, über die vorzugsweise immer offene Auslaßöffnung 44 der Kammer 38 weiter zum zweiten Anschluß 34, also aus der Inhalationseinrichtung 32 hinaus. Die Einatemluft 50 kann die Kammer 38 durch das Auslaßventil 45 nicht umströmen, da das Auslaßventil 45 geschlossen ist bzw. sich in dieser Richtung selbsttätig schließt.

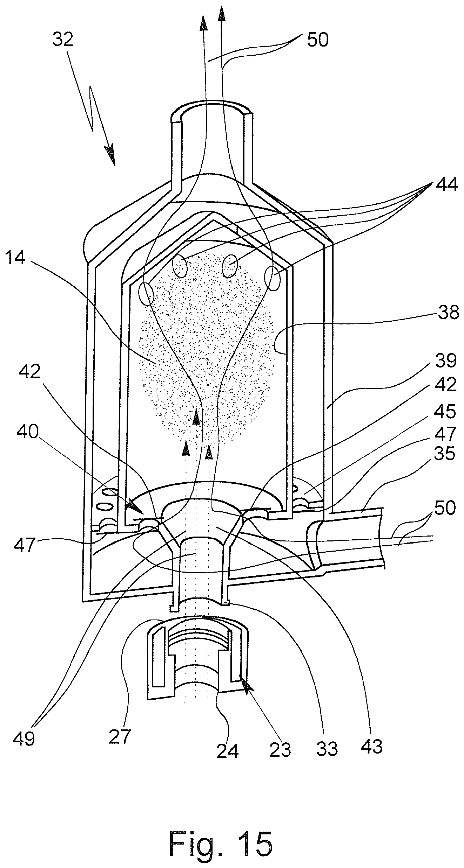

Fig. 15 zeigt eine zweite Ausführungsform der vorschlagsgemäßen Inhalationseinrichtung 32, und zwar in einer zu Fig. 14 korrespondierenden Darstellung während des Einatmens, also während der Zuführung von Atemluft zu einem nicht dargestellten Patienten. Im Gegensatz zur ersten Ausführungsform weist die Inhalationseinrichtung 32 gemäß der zweiten Ausführungsform mehrere Auslaßöffnungen 44 an der Kammer 38 auf, die insbesondere ringförmig verteilt und/oder konzentrisch bezüglich des vorzugsweise zentralen zweiten Anschlusses 34 angeordnet sind (der zweite Anschluß 34 schließt sich insbesondere axial bzw. stromab an die Ringanordnung der Auslaßöffnungen 44 an). Untersuchungen haben gezeigt, daß sich hierdurch überraschender Weise ein verlustfreier Austrag des Aerosols 14 bei guter Durchmischung mit der zugeführten oder angesaugten Atemluft bzw. Einatmenluft 50 erreichen läßt.

Es ist anzumerken, daß das Einlaßventil 40 vorzugsweise derart ausgebildet ist, daß sich deren Ventilelement 42 zur Mitte bzw. zu dem Verbindungskanal 43 hin öffnet bzw. die freie Kante oder das freie Ende zur Mitte hinweist. Insbesondere wird auf diese oder sonstige konstruktive Weise erreicht, daß die in die Kammer 38 einströmende Einatemluft 50 benachbart zum Ende des Verbindungskanals 43 einströmt, um ggf. zusätzlich ein Venturi-Effekt zu erzeugen. Jedoch sind auch andere konstruktive Lösungen möglich.

Generell ist anzumerken, daß die einzelnen Ausführungsformen und Ausführungsvarianten sowie die jeweiligen Merkmale und Aspekte auch beliebig miteinander kombiniert, aber auch unabhängig voneinander realisiert werden können.

Die vorliegende Erfindung schlägt insbesondere eine Kombination des oben beschriebenen Zerstäubers 1 oder eines sonstigen Zerstäubers 1 mit dem Adapter 23 und der Inhalationseinrichtung 32 vor. Jedoch können der Adapter 23 und die Inhalationseinrichtung 32 auch unabhängig voneinander in Kombination mit dem Zerstäuber 1 oder bei sonstigen Zerstäubern 1 verwendet werden.

Weiter ist die vorliegende Erfindung darauf gerichtet, den Adapter 23 und/oder die Inhalationseinrichtung 32 mit einer Beatmungseinrichtung bzw. für die Beatmung eines Patienten einzusetzen bzw. zu verwenden. Jedoch kann insbesondere die Inhalationseinrichtung 32 auch für sonstige Zwecke, beispielsweise als sogenannter Spacer eingesetzt werden. In diesem Fall kann der dritte Anschluß 35 entfallen oder an den Zerstäuber 1 angeschlossen werden. Bedarfsweise kann dann die Atemluft oder Zuluft über mindestens eine Zuluftöffnung 15 des Zerstäubers 1 oder auf sonstige Weise zugeführt werden. Alternativ kann der dritte Anschluß 35 auch nur zur Aufgabe von Ausatemluft 48 eingesetzt werden.

Zur Vervollständigung der Offenbarung der vorliegenden Patentanmeldung und zur bevorzugten Ausbildung des Zerstäubers 1 wird vorsorglich auf den kompletten Offenbarungsgehalt sowohl der WO 91/14468 A1 als auch der WO 97/12687 A1 verwiesen.

Im Gegensatz zu Standgeräten oder dergleichen ist der vorschlagsgemäße Zerstäuber 1 vorzugsweise transportabel ausgebildet, insbesondere handelt es sich um ein mobiles Handgerät.

Aufgrund seiner zylinderähnlichen Form und einer handlichen Größe von weniger als 9 bis 15 cm in der Länge und 2 bis 4 cm in der Breite kann der Zerstäuber 1 jederzeit vom Patienten mitgeführt werden. Der Vernebler versprüht ein definiertes Volumen der Arzneimittelzubereitung 2 unter Anwendung hoher Drücke durch kleine Düsen, so daß inhalierbare Aerosole 14 entstehen.

Der Zerstäuber 1 arbeitet insbesondere rein mechanisch. Jedoch kann der Zerstäuber 1 grundsätzlich auch auf jede sonstige Art und Weise arbeiten. Insbesondere ist der Begriff "Fördereinrichtung" bzw. "Druckerzeuger" sehr allgemein zu verstehen. Beispielsweise kann der zur Ausgabe und Zerstäubung erforderliche Druck auch durch Treibgas, eine Pumpe oder auf jede sonstige geeignete Art und Weise erzeugt werden.

Der Zerstäuber 1 ist insbesondere zur kurzzeitigen Zerstäubung der Arzneimittelzubereitung 2, beispielsweise für ein bis zwei Atemzüge, ausgebildet.

Jedoch kann er auch zur längeren oder kontinuierlichen Zerstäubung ausgebildet bzw. einsetzbar sein.

Nachfolgend werden bevorzugte Verbindungen, Bestandteile und/oder Formulierungen des Fluides bzw. der Arzneimittelzubereitung 2 aufgeführt.

Die unten genannten Verbindungen können allein oder in Kombination zur Anwendung in der erfindungsgemäßen Vorrichtung gelangen. In den unten genannten Verbindungen ist W ein pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-liydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino] ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl} -6-hydroxy-4H-benzo[1,4]oxazin-3-on
- 4-(4- {2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1 ,4]oxazin-3-on
- 1-(4-Ethoxy-carbonylamino-3-cyano-5-fluorophenyl)-2-(tert.-butylamino)ethanol
- 2-Hydroxy-5-(1 -hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl} - ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy} -propyl)-benzylsulfonamid
- 4-(2-{6-[4-(3-Cyclopentanesulfonyl-phenyl)-butoxy]-hexylamino}-1 - hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 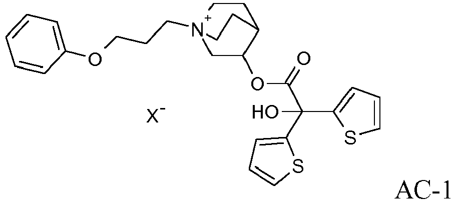 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel AC-1-en 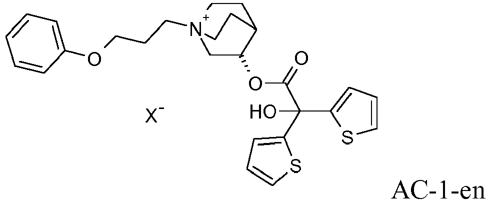 enthalten, worin X⁻ die vorstehend genannten Bedeutungen aufweisen kann. Weiterhin bevorzugte Anticholinergika sind ausgewählt aus den Salzen der Formel AC-2 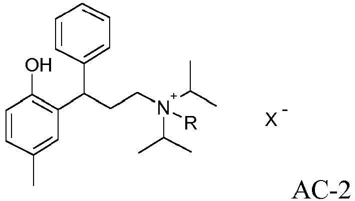 worin R entweder Methyl oder Ethyl bedeuten und worin X⁻ die vorstehend genannte Bedeutungen aufweisen kann. In einer alternativen Ausführungsform kann die Verbindung der Formel AC-2 auch in Form der freien Base AC-2-base vorliegen.

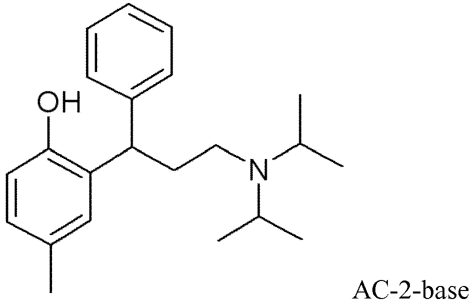

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3 S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5 -Dichloro-1 -oxo-pyridin-4-yl)-4-difluormethoxy-3 - cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2- hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1- yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6- {2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1 -yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy} -7-methoxy-chinazolin
- 4-[(3-Chlor-4-fluor-phenyl)amino]-6- {1-[(methoxymethyl)carbonyl]-piperidin-4-yloxy} -7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6- {trans-4-[(dimethylamino)sulfonylamino]-cyclohexan-1-yloxy}-7-methoxy-chinazolin
- 4-[(3-Chlor-4-fluor-phenyl)amino]-6- {trans-4-[(morpholin-4-yl)carbonylamino]-cyclohexan-1-yloxy}-7-methoxy-chinazolin
- 4-[(3-Chlor-4-fluor-phenyl)amino]-6- {trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy} -7-methoxy-chinazolin
- 4-[(3-Chlor-4-fluor-phenyl)amino]-6-(1 -aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1 -yloxy)-7-methoxy-chinazolin
- 4-[(3-Chlor-4-fluor-phenyl)amino]-6-(cis-4-{N- [(4-methyl-piperazin- 1 - yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy} -7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1 -methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy} -7-methoxy-chinazolin
- 4-[(3-Chlor-4-fluor-phenyl)amino]-6-{1-[(2-methyl-morpholin-4-yl)carbonyl]-piperidin-4-yloxy} -7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy} -7-methoxy-chinazolin
- 4-[(3-Chlor-4-fluor-phenyl)amino]-6-{1-[(3-methoxypropyl-amino)-carbonyl]-piperidin-4-yloxy} -7-methoxy-chinazolin
- 4-[(3-Chlor-4-fluor-phenyl)amino]-6-[cis-4-(N-methansulfonyl-N-methyl-amino)-cyclohexan-1 -yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan- 1 - yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4- {N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1 | Zerstäuber | 32 | Inhalationseinrichtung |
| 2 | Arzneimittelzubereitung | 33 | erster Anschluß (Inhalations-einrichtung) |
| 3 | Behälter | | |
| 4 | Beutel | 34 | zweiter Anschluß (Inhalations-einrichtung) |
| 5 | Druckerzeuger | | |
| 6 | Halterung | 35 | dritter Anschluß (Inhalations-einrichtung) |
| 7 | Antriebsfeder | | |
| 8 | Sperrelement | 36 | Ventilelement |
| 9 | Förderrohr | 37 | Vorsprung |
| 10 | Rückschlagventil | 38 | Kammer |
| 11 | Druckkammer | 39 | Gehäuse |
| 12 | Austragsdüse | 40 | Einlaßventil |
| 13 | Mundstück | 41 | Einlaß öffnung |
| 14 | Aerosol | 42 | Ventilelement |
| 15 | Zuluftöffnung | 43 | Verbindungskanal |
| 16 | erstes Gehäuseteil (Oberteil) | 44 | Auslaßöffnung |
| 17 | Innenteil | 45 | Auslaßventil |
| 17a | oberer Teil des Innenteils | 46 | Ventilöffnung |
| 17b | unterer Teil des Innenteils | 47 | Ventilelement |
| 18 | zweites Gehäuseteil (Unterteil)55 | 48 | Ausatemluft |
| 19 | Halteelement | 49 | Aerosolströmung |
| 20 | Feder (im Gehäuseunterteil) | 50 | Einatemluft |
| 21 | Behälterboden | | |
| 22 | Anstechelement | | |
| 23 | Adapter | | |
| 24 | erster Anschluß (Adapter) | | |
| 25 | Anschlußabschnitt | | |
| 26 | Stutzen | | |
| 27 | zweiter Anschluß (Adapter) | | |
| 28 | dritter Anschluß (Adapter) | | |
| 29 | Schlauch | | |
| 30 | Ringkanal | | |
| 31 | Sperrventil | | |

## Patentansprüche

1. Inhalationseinrichtung (32) mit einer Kammer (38) zur Zwischenspeicherung eines Aerosols (14), mit einem ersten Anschluß (33) für einen das Aerosol (14) erzeugenden Zerstäuber (1) und einem zweiten, patientenseitigen Anschluß (34) zur Abgabe des Aerosols (14) sowie mit einem dritten Anschluß (35) für Atemluft,
wobei die Kammer (38) einlaßseitig mit dem ersten Anschluß (33) zumindest bei angeschlossenem Zerstäuber (1) ventilfrei fluidisch verbunden und parallel einlaßseitig über ein Einlaßventil (40) an den dritten Anschluß (35) angeschlossen ist, so daß Atemluft vom dritten Anschluß (35) in die Kammer (38) strömen kann, und
wobei der zweite Anschluß (34) über ein Auslaßventil (45) parallel zur Kammer (38) an den dritten Anschluß (35) angeschlossen ist, so daß Atemluft vom zweiten Anschluß (34) an der Kammer (38) vorbei über das Auslaßventil (45) zum dritten Anschluß (35) strömen kann,
**dadurch gekennzeichnet,**
**daß** zwischen der Kammer (38) bzw. einer die Kammer (38) bildenden Wandung einerseits und einem Gehäuse (39) andererseits ein Ringraum ausgebildet ist, durch den die Atemluft vom zweiten Anschluß (34) über das Auslaßventil (45) zum dritten Anschluß (35) strömen kann.

2. Inhalationsvorrichtung (32) nach Anspruch 1, **dadurch gekennzeichnet daß** die Kammer (38) auslaßseitig mehrere zumindest im wesentlichen ringförmig angeordnete Auslaßöffnungen (44) aufweist.

3. Inhalationseinrichtung nach Anspruch 1 oder 2, **dadurch gekennzeichnet, daß** die Kammer (38) auslaßseitig ventilfrei fluidisch mit dem zweiten Anschluß (34) verbunden ist.

4. Inhalationseinrichtung nach einem der Ansprüche 1 bis 3, **dadurch gekennzeichnet, daß** das Einlaßventil (40) als Einweg- oder Rückschlagventil ausgebildet ist.

5. Inhalationseinrichtung nach einem der Ansprüche 1 bis 4, **dadurch gekennzeichnet, daß** das Auslaßventil (45) als Einweg- oder Rückschlagventil ausgebildet ist.

6. Inhalationsvorrichtung nach einem der Ansprüche 1 bis 5, **dadurch gekennzeichnet, daß** das Auslaßventil (45) mehrere Ventilöffnungen (46) aufweist, wobei die Ventilöffnungen (46) durch ein gemeinsames Ventilelement (47) oder mehrere separate Ventilelemente (47) verschließbar sind.

7. Inhalationsvorrichtung nach Anspruch 6, **dadurch gekennzeichnet, daß** die Ventilöffnungen (46) ringförmig über einen Umfang des Ringraums verteilt angeordnet sind.

8. Inhalationsvorrichtung nach Anspruch 6 oder 7, **dadurch gekennzeichnet, daß** das Auslaßventil (45) bzw. dessen Ventilelement(e) (47) selbstschließend ausgebildet und/oder in die Schließstellung vorgespannt ist.

9. Inhalationseinrichtung nach einem der Ansprüche 1 bis 8, **dadurch gekennzeichnet, daß** der zweite Anschluß (34) als Mundstück (13) oder zum Anschluß eines Schlauchs (29) ausgebildet ist.

10. Inhalationseinrichtung nach einem der Ansprüche 1 bis 9, **dadurch gekennzeichnet, daß** der dritte Anschluß (35) zur Verbindung mit einer Beatmungseinrichtung, insbesondere einem Beatmungsschlauch, ausgebildet ist.

11. Zerstäuber mit einer Inhalationseinrichtung (32) nach einem der Ansprüche 1 bis 10.

## Claims

1. Inhalation device (32) having
a chamber (38) for the intermediate storage of an aerosol (14),
a first connection (33) for a nebuliser (1) that produces the aerosol (14) and
a second connection (34) on the patient side for delivering the aerosol (14) and
a third connection (35) for breathable air,
wherein the chamber (38) is fluidically connected in valve-free manner on the inlet side to the first connection (33) at least when the nebuliser (1) is attached and is connected to the third connection (35) in parallel on the inlet side via an inlet valve (40) so that breathable air from the third connection (35) can flow into the chamber (38), and
wherein the second connection (34) is connected to the third connection (35) via an outlet valve (45) parallel to the chamber (38), so that breathable air can flow from the second connection (34) past the chamber (38) through the outlet valve (45) to the third connection (35),
**characterised in that**
an annular chamber is formed between the chamber (38) or a wall that forms the chamber (38), on the one hand, and a housing (39), on the other hand, said annular chamber allowing the breathable air to flow from the second port (34) through the outlet valve (45) to the third port (35).

2. Inhalation device (32) according to claim 1, **characterised in that** the chamber (38) comprises on the outlet side a plurality of outlet openings (44) arranged at least substantially in an annular configuration.

3. Inhalation device according to claim 1 or 2, **characterised in that** the chamber (38) is fluidically connected to the second connection (34) on the outlet side in valve-free manner.

4. Inhalation device according to one of claims 1 to 3, **characterised in that** the inlet valve (40) is embodied as a one-way or non-return valve.

5. Inhalation device according to one of claims 1 to 4, **characterised in that** the outlet valve (45) is embodied as a one-way or non-return valve.

6. Inhalation device according to one of claims 1 to 5, **characterised in that** the outlet valve (45) comprises a plurality of valve openings (46), wherein the valve openings (46) can be closed off by a common valve element (47) or a plurality of separate valve elements (47).

7. Inhalation device according to claim 6, **characterised in that** the valve openings (46) are arranged in a ring around a periphery of the annular chamber.

8. Inhalation device according to claim 6 or 7, **characterised in that** the outlet valve (45) or its valve element(s) (47) is embodied to be self-closing and/or biased into the closed position.

9. Inhalation device according to one of claims 1 to 8, **characterised in that** the second connection (34) is embodied as a mouthpiece (13) or for connection of a tube (29).

10. Inhalation device according to one of claims 1 to 9, **characterised in that** the third connection (35) is configured for connection to a ventilator, particularly a ventilating tube.

11. Nebuliser having an inhalation device (32) according to one of claims 1 to 10.

## Revendications

1. Dispositif d'inhalation (32) avec une chambre (38) pour le stockage intermédiaire d'un aérosol (14), avec un premier raccord (33) pour un pulvérisateur (1) générant l'aérosol (14) et un deuxième raccord (34) côté patient pour l'émission de l'aérosol (14) ainsi qu'avec un troisième raccord (35) pour de l'air inhalé, dans lequel la chambre (38) est reliée fluidiquement sans soupape côté entrée au premier raccord (33) au moins pour un pulvérisateur (1) raccordé et est raccordée parallèlement côté entrée par le biais d'une soupape d'entrée (40) au troisième raccord (35) de sorte que de l'air inhalé puisse s'écouler du troisième raccord (35) dans la chambre (38), et
dans lequel le deuxième raccord (34) est raccordé par le biais d'une soupape de sortie (45) parallèlement à la chambre (38) au troisième raccord (35) de sorte que de l'air inhalé puisse s'écouler du deuxième raccord (34) en passant devant la chambre (38) par le biais de la soupape de sortie (45) au troisième raccord (35),
**caractérisé en ce**
**qu'**un espace annulaire est réalisé entre la chambre (38) ou une paroi formant la chambre (38) d'une part et un boîtier (39) d'autre part, à travers lequel l'air inhalé peut s'écouler du deuxième raccord (34) par le biais de la soupape de sortie (45) au troisième raccord (35).

2. Dispositif d'inhalation (32) selon la revendication 1, **caractérisé en ce que** la chambre (38) présente côté sortie plusieurs ouvertures de sortie (44) agencées au moins sensiblement en forme d'anneau.

3. Dispositif d'inhalation selon la revendication 1 ou 2, **caractérisé en ce que** la chambre (38) est reliée côté sortie sans soupape fluidiquement au deuxième raccord (34).

4. Dispositif d'inhalation selon l'une des revendications 1 à 3, **caractérisé en ce que** la soupape d'entrée (40) est réalisée comme soupape à une voie ou antiretour.

5. Dispositif d'inhalation selon l'une des revendications 1 à 4, **caractérisé en ce que** la soupape de sortie (45) est réalisée comme soupape à une voie ou antiretour.

6. Dispositif d'inhalation selon l'une des revendications 1 à 5, **caractérisé en ce que** la soupape de sortie (45) présente plusieurs ouvertures de soupape (46), dans lequel les ouvertures de soupape (46) sont refermables par un élément de soupape (47) commun ou plusieurs éléments de soupape (47) séparés.

7. Dispositif d'inhalation selon la revendication 6, **caractérisé en ce que** les ouvertures de soupape (46) sont agencées de manière répartie en anneau sur une périphérie de l'espace annulaire.

8. Dispositif d'inhalation selon la revendication 6 ou 7, **caractérisé en ce que** la soupape de sortie (45) ou son/ses élément(s) de soupape (47) est/sont réalisé(s) à fermeture automatique et/ou est/sont précontraint(s) dans la position de fermeture.

9. Dispositif d'inhalation selon l'une des revendications 1 à 8, **caractérisé en ce que** le deuxième raccord (34) est réalisé comme embout (13) ou pour le raccordement d'un tuyau (29).

10. Dispositif d'inhalation selon l'une des revendications 1 à 9, **caractérisé en ce que** le troisième raccord (35) est réalisé pour la liaison avec un dispositif respiratoire, en particulier un tuyau respiratoire.

11. Pulvérisateur avec un dispositif d'inhalation (32) selon l'une des revendications 1 à 10.
